(19) Europäisches Patentamt

European Patent Office

Office européen des brevets

(11) Veröffentlichungsnummer: **0 466 042 A2**

## (12) EUROPÄISCHE PATENTANMELDUNG

(21) Anmeldenummer: **91111193.8**

(22) Anmeldetag: **05.07.91**

(51) Int. Cl.5: **C12P 17/12**, C12P 17/00,
//(C12P17/00,C12R1:38)

(30) Priorität: **06.07.90 CH 2272/90**
**01.10.90 CH 3149/90**

(43) Veröffentlichungstag der Anmeldung:
**15.01.92 Patentblatt 92/03**

(84) Benannte Vertragsstaaten:
**AT BE CH DE DK ES FR GB IT LI NL SE**

(71) Anmelder: **LONZA AG**
**Gampel/Wallis Geschäftsleitung Basel**
**CH-4002 Basel(CH)**

(72) Erfinder: **Hoeks, Frans**
**Dammweg 11A**
**Naters (Kanton Wallis)(CH)**

(74) Vertreter: **Weinhold, Peter, Dr. et al**
**Patentanwälte Dipl.-Ing. G. Dannenberg Dr.**
**P. Weinhold Dr. D. Gudel Dipl.-Ing. S.**
**Schubert Dr. P. Barz Siegfriedstrasse 8**
**W-8000 München 40(DE)**

(54) **Mikrobiologisches Verfahren zur Oxidation von Methylgruppen.**

(57) Beschrieben wird ein mikrobiologisches Verfahren zur Oxidation von Methylgruppen in aromatischen 5- oder 6-Ring-Heterocyclen zur entsprechenden Carbonsäure.

Die Umsetzung des Heterocyclus erfolgt mittels Toluol-, Xylol- oder Cymol-verwertenden Mikroorganismen der Gattung Pseudomonas, wobei ein Induktor, der aromatische Heterocyclus, als Substrat, und eine Kohlenstoff- und Energiequelle zugeführt werden und nach Erreichen der maximalen Produktkonzentration das Produkt abgetrennt wird.

EP 0 466 042 A2

Die Erfindung betrifft ein mikrobiologisches Verfahren zur Oxidation von Methylgruppen an aromatischen 5- oder 6-Ring-Heterocyclen zur entsprechenden Carbonsäure, wobei der Heterocyclus keinen Substituenten am benachbarten Kohlenstoffatom der zu oxidierenden Methylgruppe aufweist.

Diese Carbonsäurederivate können beispielsweise als Zwischenprodukte für weitere chemische Synthesen verwendet werden. Z.B. ist 2-Pyrazincarbonsäure ein wichtiges Zwischenprodukt zur Herstellung des Tuberkulostatikums Pyrazinamid (2-Pyrazincarbonsäureamid) (Römpps Chemie Lexikon, Bd. 5, 1987, S.3411).

Eingehende Untersuchungen zur mikrobiologischen Herstellung von Carbonsäuren wurden bis jetzt mit aromatischen Kohlenwasserstoffen durchgeführt.

Die Produktion von Carbonsäuren durch mikrobiologische Oxidation von methylierten Aromaten wurde ausführlich in den Arbeiten von Raymond und Mitarbeitern beschrieben (Raymond et al, Process Biochem., 1969, S.71-74).

Die US-PS 3 383 289 beschreibt ein Verfahren zur biochemischen Oxidation von Methylgruppen in aromatischen Kohlenwasserstoffen mit einem gram-positiven Mikroorganismenstamm der Gattung Nocardia.

Nachteile dieser Verfahren sind, dass beispielsweise bei der Methylgruppenoxidation von aromatischen Kohlenwasserstoffen der Benzolring der entsprechenden Säure gespalten wird.

Von Pseudomonas putida ATCC 33015 ist bekannt, dass die biochemische Oxidation der Methylgruppen von Toluol in 3 Schritten zur Benzoesäure verläuft. Durch Einwirkung der Toluol-monoxygenase entsteht zuerst Benzylalkohol, welcher dann in zwei weiteren Schritten, katalysiert durch eine Alkohol- und eine Aldehyddehydrogenase, zur Säure überführt wird.

In diesem Stamm liegen sowohl die Xyl-Gene, die für Enzyme des Xylol-Abbaus kodieren, als auch die Gene, welche für die Regulation der Xyl-Gene verantwortlich sind, auf dem Plasmid pWWO. Dieses archaetypische Tol-Plasmid wurde molekularbiologisch bereits eingehend untersucht (Harayama et al, J.Bacteriol. 171, 1989, S.5048-5055; Burlage et al, Appl.Environ Microbiol. 55, 1989, S.1323-1328).

Ebenso sind aus der Literatur mikrobiologische Verfahren zur Oxidation von Methylgruppen eines N-Heterocyclus bekannt. Nach der SU-PS 417 468 wird 2-Methylpyridin mit einem gram-positiven Mikroorganismenstamm der Gattung Nocardia zur entsprechenden Säure oxidiert.

Die SU-PS 228 688 beschreibt ein mikrobiologisches Verfahren zur Herstellung von Nikotinsäure aus 3-Methylpyridin mit einem gram-positiven Mikroorganismus der Gattung Mycobakterium.

Aus der SU-PS 302 341 ist ein mikrobiologisches Verfahren zur Herstellung von Nikotinsäure mit gram-positiven Bakterien der Gattung Nocardia bekannt.

Die Nachteile der Methylgruppenoxidation von N-Heterocyclen mit gram-positiven Bakterien bestehen darin, dass bei diesen Alkan-verwertenden Bakterien das Mischungsverhältnis des Alkans zur oxidierenden Substanz genau abgestimmt sein muss, um eine Biotransformation zu erreichen, und dass keine Biotransformation des Substrats in Abwesenheit des Alkans stattfindet, d.h., das zur Induktion verwendete Alkan muss immer, auch bei der Umsetzung des Substrats, anwesend sein. Durch Vergleichsversuche mit dem gram-positiven Bakterium Nocardia und unserem gram-negativen Pseudomonas konnte eindeutig gezeigt werden, dass Nocardia sogar in Gegenwart eines Alkans, wie z.B. Dodecan, 3-Methylpyridin nicht zur entsprechenden Nikotinsäure oxidiert.

Des weiteren beschreibt die US-PS 4 859 592 ein Verfahren zur Herstellung von Picolinsäure mit Pseudomonas putida, indem ein Alkyl-substituierter aromatischer Kohlenwasserstoff in Gegenwart von molekularem Sauerstoff in einer ersten Stufe durch eine Dioxygenase ein 2-Hydroxymuconsäuresemialdehyd gebildet wird, dieser dann in einer zweiten Stufe mit Ammoniak oder einem primären Amin zur entsprechenden Nikotinsäure umgesetzt wird.

Der Nachteil dieses Verfahrens besteht darin, dass die entsprechende Picolinsäure erst in der zweiten Stufe durch die Umsetzung des 2-Hydroxymuconsäuresemialdehyds mit Ammoniak gebildet wird.

Ein mikrobiologisches Verfahren zur Oxidation von Methylgruppen an Heterocyclen ist ebenso in der europäischen Patentanmeldung Nr. 91 101 902.4 beschrieben.

In diesem Verfahren werden die Mikroorganismen zunächst in einem Kulturmedium, beispielsweise mit p-Xylol als einzige Kohlenstoff- und Energiequelle, angezüchtet, danach abgetrennt und anschliessend wird die Biotransformation durch die Zugabe des Eduktes durchgeführt. Durch Erschöpfung der Biomasseaktivität werden bei diesem zweistufigen Verfahren relativ geringe Produktkonzentrationen erreicht.

Die Aufgabe der vorliegenden Erfindung ist es, die Nachteile dieser Verfahren zu vermeiden und ein Verfahren zu entwickeln, das im grosstechnischen Massstab mit hohen Raumzeitausbeuten durchgeführt werden kann, bei dem eine viel höhere Produktkonzentration erreicht wird und die Carbonsäuren nach der Isolation in hoher Ausbeute anfallen.

Die Aufgabe konnte gelöst werden mit einem Verfahren nach Patentanspruch 1, dadurch dass in ein Kulturmedium, enthaltend Toluol-, Xylol- oder Cymol-verwertende Mikroorganismen der Gattung Pseudomonas,

a) ein Induktor,

b) der methylierte aromatische 5- oder 6-Ring-Heterocyclus, als Substrat für die Biotransformation, und

c) eine oder mehrere Kohlenstoff- und Energiequellen

zugeführt werden und dass nach Erreichen der maximalen Produktkonzentration die Carbonsäure abgetrennt wird.

Die Umsetzung kann erfindungsgemäss mit Toluol-, Xylol- oder Cymol-verwertenden Mikroorganismen der Gattung Pseudomonas, vorzugsweise der Spezies Pseudomonas putida, insbesondere mit dem Xylol-verwertende Mikroorganismenstamm Pseudomonas putida, hinterlegt bei der American Type Culture Collection, 12301 Parklawn Drive Rockville, Maryland 20852, USA, unter der Nummer ATCC 33015 durchgeführt werden.

Für das Verfahren ebenso geeignet sind Mutanten dieser Mikroorganismen sowie andere Mikroorganismen, in die entweder durch Konjugation oder durch gentechnologische Methoden das für die Umsetzung notwendige Tol-Plasmid eingebracht wurde.

Das erfindungsgemässe Verfahren zur Oxidation von Methylgruppen in aromatischen 5- oder 6-Ring-Heterocyclen wird zweckmässig derart durchgeführt, dass man in dem ersten Verfahrensschritt a) gemäss Patentanspruch 4 oder 5, in einer sogenannten Batch-Phase, produktionsfähige Biomasse erzeugt. Dazu werden die Mikroorganismen auf bekannte Weise entsprechend der europäischen Patentanmeldung Nr. 91 101 902.4 entweder mit Vertretern der Verbindungsreihe Toluol, Xylol und seine Isomere oder Cymol und seine Isomere, wie p-Xylol, m-Xylol, p-Cymol, oder mit m-Cymol als Induktor und einzige Kohlenstoff- und Energiequelle in einem Mineralmedium wie z.B. Kulla et al, Arch. Microbiol. 135, 1983, S.1-7, angezogen oder sie werden mit Vertretern der Verbindungsreihe Toluol, Xylol und seiner Isomere oder Cymol und seiner Isomere, wie p-Xylol, m-Xylol, p-Cymol oder mit m-Cymol als Induktor in einem Komplexmedium wie z.B. "Nutrient Broth Nr.2", Oxoid Ltd., England, oder in einem Mineralmedium wie z.B. Kulla et al, Arch. Microbiol. 135, 1983, S.1-7 mit Kohlenstoffquellen wie Kohlehydraten, Zuckeralkoholen, niedrig siedenden aliphatischen Alkoholen, aliphatischen Fettsäuren oder Aminosäuren als Kohlenstoff- und Energiequelle angezogen.

Die Verbindungen, die dem Mikroorganismus als Kohlenstoff- und Energiequelle dienen, wie p-Xylol, m-Xylol, p-Cymol, m-Cymol oder Toluol, dienen zweckmässig ebenfalls zur Induktion der für die Umsetzung verantwortlichen Enzyme des Mikroorganismus. Diese Enzyminduktion kann auch mit Verbindungen durchgeführt werden, die dem Mikroorganismus nicht als Kohlenstoff- und Energiequelle dienen, wie beispielsweise mono- und disubstituierte Methyl-, Ethyl- und Chlortoluole, Benzylalkohole und p-Chlorbenzaldehyd, o-Xylol, o-Cymol, die schon als Enzyminduktoren für den Abbau aromatischer Kohlenwasserstoffe beschrieben sind (Abril M.A. et al, J.Bacteriol., Vol.171, 1989, S.6782-6789). Diese Verbindungen werden zweckmässig beispielsweise gasförmig, gemäss den Angaben von Hosler u. Eltz (Microbiol.Conversion of p-Xylene in stirred Fermenters, Fermentation Advances, S.789-805, Acad.Press.Inc. NY 1969), zugeführt. Die zur Induktion verwendeten Verbindungen können auch flüssig zugeführt werden.

Zweckmässig wird der Induktor so zugegeben, dass die spezifische Induktorabbaurate zwischen 0,01 und 50 mmol/g Trockenmasse/Stunde liegt.

Vorzugsweise werden die Mikroorganismen in einem Mineralmedium mit p-Xylol kultiviert, bis die Zellsuspension 0,01 bis 200 g Trockenmasse enthält.

Zweckmässig werden die Mikroorganismen bei einem pH-Wert von 5 bis 9, vorteilhaft bei einem pH-Wert von 7 bis 8, kultiviert.

Die Kultivierung wird in allen Verfahrensschritten zweckmässig bei einer Temperatur von 15 bis 90 °C, vorzugsweise bei 25 bis 35 °C, durchgeführt.

Mit der gezüchteten Biomasse können weitere Kulturen beimpft werden, die dann zweckmässig die gleiche Zusammensetzung haben.

Beim erfindungsgemässen Verfahren ist die gezüchtete Biomasse Ausgangspunkt für die mikrobiologische Oxidation der Methylgruppe in den Heterocyclen in der sogenannten Biotransformationsphase, die kontinuierlich oder diskontinuierlich durchgeführt werden kann.

Nach der Biomasseanzüchtung kann b) die Zufuhr des Induktors gemäss Patentanspruch 4 oder 5 unterbunden werden.

Die Verfahrensschritte c) bis e) gemäss Patentanspruch 4 ozw. c) bis f) gemäß Patentanspruch 5 kennzeichnen bei diesem Verfahren die sogenannte Biotransformationsphase.

Das Kulturmedium und die Kulturbedingungen in der Biotransformationsphase ist dem der Biomassekultivierung weitgehend ähnlich.

Die Biotransformationsphase ist dadurch charakterisiert, dass

c) der Kulturlösung anschliessend das Edukt, den methylierten aromatischen 5- oder 6-Ring-Heterocyclus und gegebenenfalls die Kohlenstoff- und Energiequelle hinzugefügt wird, bis die Mikroorganismen eine Abnahme der Aktivität für die Biotransformation aufweisen,

d) dann die Aktivität der Mikroorganismen durch Zufuhr des Induktors, gegebenenfalls noch durch gleichzeitige Unterbindung der Kohlenstoff- und Energiequelle, regeneriert wird, bis die spezifische Induktorabbaurate zwischen 0,01 und 50 mmol/g Trockenmasse/Stunde beträgt, und

e) die Verfahrensschritte b) bis d) wiederholt werden, damit man die Biotransformation über längere Zeiträume durchführen kann und damit die maximale Produktkonzentration und/oder einen kontinuierlichen Produktionsprozess verwirklicht und hohe Raumzeitausbeuten erreicht.

Das Edukt, der Heterocyclus, wird zweckmässig so zugeführt, dass die Konzentration im Kulturmedium zwischen 0,0001 und 5% liegt, vorzugsweise zwischen 0,01 und 1%.

Als Kohlenstoff- und Energiequelle können die in der Fachwelt üblichen und z.B. für Pseudomonas-Stämme literaturbekannten Verbindungen eingesetzt werden (The Prokaryotes, Ed. by M.P. Starr, H.Stolp, H.G.Trüper, A.Balow, H.G.Schlegel, Springer Verlag 1981). Zweckmässige Kohlenstoff- und Energiequellen sind Kohlenhydrate, Zuckeralkohole, niedrig siedende aliphatische Alkohole, aliphatische Fettsäuren oder Aminosäuren. Vorzugsweise wird Glucose und/oder Glycerin und/oder Glutamat angewendet. Diese Kohlenstoff- und Energiequelle wird zweckmässig so zugeführt, dass die spezifische Substratabbaurate zwischen 0,01 und 200 mmol/g Trockenmasse/Stunde liegt.

Nach Kultivierung in einer diskontinuierlich durchgeführten Biotransformationsphase von in der Regel nach 10 bis 250 h kann eine Produktkonzentration in der Kultur von mehr als 100 mmol/l erreicht werden.

Nach einer Modifikation des Verfahrens ist es auch möglich, nach Beendigung der Fermentation einen Teil der Kulturlösung abzulassen und mit dem verbleibenden Teil, aufgefüllt mit neuem Kulturmedium, eine erneute Biotransformation-Kultivierung anzufahren. Mit dem sogenannten repeated-Biotransformations-Verfahren gelingt es, die volumetrische Produktivität der Fermentation zu erhöhen. Die volumetrische Produktivität kann noch weiter erhöht werden, indem man gleichzeitig neues Medium hinzuführt und die Kulturlösung ablässt (kontinuierliches Verfahren).

Eine Entlastung der Kultur kann dadurch erreicht werden, dass man das Edukt (den Heterocyclus) mittels Ionenaustauscher oder mittels Elektrodialyse entsalzt und reinigt.

Die Gewinnung der Produkte aus der Kulturlösung geschieht durch übliche Methoden und kann zweckmässig kontinuierlich oder diskontinuierlich durchgeführt werden, wobei die kontinuierliche Gewinnung bevorzugt ist. Beispielsweise kann die Abtrennung der Biomasse durch z.B. Zentrifugieren, Ultrafiltration oder Mikrofiltration durchgeführt werden.

Die sowohl bei der Elektrodialyse wie auch mittels Ionenaustauscher anfallenden konzentrierten Produktlösungen können entweder durch Verdampfen oder auch durch Umkehrosmose aufkonzentriert und anschliessend azeotrop entwässert werden.

Wird das Produkt kontinuierlich mittels Elektrodialyse (z.B. gemäss Eymondt u. Wandrey, 1990, Chem.Ing.Tech. 62, Nr.2, S.134-135) gewonnen, kann die Produktivität noch weiter erhöht werden.

Zweckmässig wird nach diesem Verfahren 5-Methyl-2-pyrazincarbonsäure oder 6-Methyl-2-pyrazincarbonsäure, insbesondere 5-Methyl-2-pyrazincarbonsäure hergestellt.

Beispiel 1

Herstellung von 5-Methyl-2-pyrazincarbonsäure

Pseudomonas putida ATCC 33015 wurde in einem Mineralmedium (Kulla et al, Arch.Microbiol. 135, 1983, S.1-7) mit p-Xylol als einzige Kohlenstoff- und Energiequelle in einem Fermenter bei pH 7,0 und einer Temperatur von 30°C angezogen. Der Enzyminduktor p-Xylol wurde gasförmig gemäss den Angaben von Hasler und Eltz (Microbiol.Conversion of p-Xylene in Stirred Fermenters", Fermentation Advances, pp.789-805, Acad.Press.Inc. NY 1969) zugeführt, bis eine Biomassekonzentration von 7,8 g Trockenmasse/l erreicht wurde. Anschliessend wurde die Zufuhr des Induktors unterbunden und 2,5-Dimethylpyrazin und eine 50%ige Glucoselösung mit einer Geschwindigkeit von 1 g/l/h zum Fermenter zudosiert. Nach 4 h, wenn die Biotransformationsaktivität der Biomasse auf ca. 80% abgesunken war, wurde wieder p-Xylol (2 h) bis zu einer spezifischen Induktorabbaurate von 0,51 mmol/g Trockenmasse/h zudosiert, unter gleichzeitiger Unterbindung der Glucosezudosierung. Das Absinken der Biotransformationsaktivität der Biomasse wurde anhand des Verbrauchs der Lauge festgestellt, die für die Neutralisation des Produktes verbraucht worden war. Nachher wurde alternierend Glucose und p-Xylol wie beschrieben zudosiert, bis die Konzentration der 5-Methyl-2-pyrazincarbonsäure einen Wert von 101 mmol/l erreicht hatte, was einer Ausbeute von 95% entsprach, bezogen auf eingesetztes 2,5-Dimethylpyrazin.

Beispiel 2

Herstellung von 6-Methyl-2-pyrazincarbonsäure

Pseudomonas putida ATCC 33015 wurde analog Beispiel 1 angezüchtet, bis die Biomassekonzentration von 7,8 g Trockenmasse/l erreicht wurde.

Anschliessend wurde die Zufuhr des Induktors unterbunden und 2,6-Dimethylpyrazin und eine 50%ige Glucoselösung mit einer Geschwindigkeit von 1 g/l/h zum Fermenter zudosiert. Nach 4 h, wenn die Biotransformationsaktivität der Biomasse auf ca. 75% abgesunken war, wurde wieder p-Xylol (2 h) bis zu einer spezifischen Induktorabbaurate von 0,45 mmol/g Trockenmasse/h zudosiert und die Glucosezugabe unterbunden. Das Absinken der Biotransformationsaktivität der Biomasse wurde anhand des Verbrauchs der Lauge festgestellt, die für die Neutralisation des Produktes verbraucht worden war.

Nachher wurde alternierend Glucose und p-Xylol wie beschrieben zudosiert, bis die Konzentration der 6-Methyl-2-pyrazincarbonsäure einen Wert von 105 mmol/l erreicht hatte, was einer Ausbeute von 95% entsprach, bezogen auf eingesetztes 2,6-Dimethylpyrazin.

## Patentansprüche

1. Mikrobiologisches Verfahren zur Oxidation von Methylgruppen an aromatischen 5- oder 6-Ring-Heterocyclen, wobei der Heterocyclus keinen Substituenten am benachbarten Kohlenstoffatom der zu oxidierenden Methylgruppe aufweist, zur entsprechenden Carbonsäure, dadurch gekennzeichnet, dass einem Kulturmedium, enthaltend Toluol-, Xylol- oder Cymol-verwertende Mikroorganismen der Gattung Pseudomonas,

   a) ein Induktor,
   b) der methylierte aromatische 5- oder 6-Ring-Heterocyclus als Substrat für die Biotransformation und gegebenenfalls
   c) eine oder mehrere Kohlenstoff- und Energiequellen zugeführt werden und dass nach Erreichen der maximalen Produktionskonzentration die Carbonsäure abgetrennt wird.

2. Verfahren nach Patentanspruch 1, dadurch gekennzeichnet, dass man als Induktor Verbindungen einsetzt, die dem Mikroorganismus als Kohlenstoff- und Energiequelle dienen, wie ein oder mehrere Vertreter aus der Verbindungsreihe Toluol, Xylol oder seine Isomere oder Cymol oder seine Isomere.

3. Verfahren nach Patentanspruch 1, dadurch gekennzeichnet, dass man als Induktor Verbindungen einsetzt, die dem Mikroorganismus nicht als Kohlenstoff- und Energiequelle dienen.

4. Verfahren nach Patentansprüchen 1 und 2, dadurch gekennzeichnet, daß man den Prozeß kontinuierlich durchführt, indem man

   a) die Mikroorganismen mit dem Induktor anzüchtet, bis genügend Zellen für die Produktion vorhanden sind,
   b) dann die Zufuhr des Induktors unterbindet,
   c) anschliessend den methylierten aromatischen 5- oder 6-Ring-Heterocyclus und gegebenenfalls die Kohlenstoff- und Energiequelle hinzufügt, bis die Mikroorganismen eine Abnahme der Aktivität für die Biotransformation aufweisen,
   d) dann die Aktivität der Mikroorganismen durch Zufuhr des Induktors, gegebenenfalls noch durch gleichzeitige Unterbindung der Kohlenstoff- und Energiequelle, regeneriert, und
   e) die Verfahrensschritte b) bis d) wiederholt.

5. Verfahren nach den Ansprüchen 1 und 2, dadurch gekennzeichnet, daß man den Prozeß diskontinuierlich durchführt, indem man

   a) die Mikroorganismen mit dem Induktor anzüchtet, bis genügend Zellen für die Produktion vorhanden sind,
   b) dann die Zufuhr des Induktors unterbindet,
   c) anschliessend den methylierten aromatischen 5- oder 6-Ring-Heterocyclus und
   d) gegebenenfalls die Kohlenstoff- und Energiequelle hinzufügt, bis die Mikroorganismen eine Abnahme der Aktivität für die Biotransformation aufweisen,
   e) dann die Aktivität der Mikroorganismen durch Zufuhr des Induktors, gegebenenfalls noch durch gleichzeitige Unterbindung der Kohlenstoff- und Energiequelle, regeneriert, und
   f) die Verfahrensschritte b), d) und e) wiederholt.

6. Verfahren nach mindestens einem der Patentansprüche 1, 2, 4 und 5, dadurch gekennzeichnet, dass der Induktor so zugegeben wird, dass die spezifische Induktorabbaurate zwischen 0,01 und 50 mmol/g Trockenmasse/Stunde liegt.

7. Verfahren nach mindestens einem der Patentansprüche 1 bis 5, dadurch gekennzeichnet, dass der methylierte aromatische 5- oder 6-Ring-Heterocyclus so zugegeben wird, dass die Konzentration im Kulturmedium zwischen 0,0001 und 5% liegt.

**8.** Verfahren nach mindestens einem der Patentansprüche 1 bis 5, dadurch gekennzeichnet, dass die Kohlenstoff- und Energiequelle so zugegeben wird, dass die spezifische Substratabbaurate zwischen 0,01 und 200 mmol/g Trockenmasse/Stunde liegt.

**9.** Verfahren nach mindestens einem der Patentansprüche 1 bis 5 und 8, dadurch gekennzeichnet, dass als Kohlenstoff- und Energiequelle Kohlenhydrate, Zuckeralkohole, niedrig siedende aliphatische Alkohole, aliphatische Fettsäuren oder Aminosäuren eingesetzt werden.

**10.** Verfahren nach mindestens einem der Patentansprüche 1 bis 9, dadurch gekennzeichnet, dass das Produkt kontinuierlich oder diskontinuierlich abgetrennt wird.

**11.** Verfahren nach mindestens einem der Patentansprüche 1 bis 10, dadurch gekennzeichnet, dass man die Umsetzung mittels Toluol-, Xylol- oder Cymol-verwertenden Mikroorganismen der Spezies Pseudomonas putida durchführt.

**12.** Verfahren nach mindestens einem der Patentansprüche 1 bis 11, dadurch gekennzeichnet, dass man die Umsetzung mit dem Xylol-verwertenden Mikroorganismenstamm Pseudomonas putida mit der Bezeichnung ATCC 33015 oder einer wirksamen Mutante von diesem durchführt.

**13.** Verfahren nach Patentanspruch 1 bis 12 zur Herstellung von 5-Methyl-2-pyrazincarbonsäure, dadurch gekennzeichnet, dass man als Substrat 2,5-Dimethylpyrazin einsetzt.

**14.** Verfahren nach Patentansprüchen 1 bis 12 zur Herstellung von 6-Methyl-2-pyrazincarbonsäure, dadurch gekennzeichnet, dass man als Substrat 2,6-Dimethylpyrazin einsetzt.